Europäisches Patentamt

(19) European Patent Office

Office européen des brevets

(11) Publication number: **0 401 700**
**A2**

# EUROPEAN PATENT APPLICATION

(21) Application number: **90110465.3**

(22) Date of filing: **01.06.90**

(51) Int. Cl.⁵: **C12N 1/20, C12P 19/04,**
**C11D 3/37**

The applicant has filed a statement in accordance with Rule 28 (4) EPC (issue of a sample only to an expert). Accession number(s) of the deposit(s): FERM BP-2905.

(30) Priority: **05.06.89 JP 142421/89**

(43) Date of publication of application:
**12.12.90 Bulletin 90/50**

(84) Designated Contracting States:
**AT BE CH DE ES FR GB IT LI NL SE**

(71) Applicant: **LION CORPORATION**
**3-7, Honjo 1-chome**
**Sumida-ku Tokyo(JP)**

(72) Inventor: **Tanaka, Yoshimasa**
**1-3-24, Fujimigaoka, Nimomiya-cho**
**Naka-gun, Kanagawa-ken(JP)**
Inventor: **Fukui, Tomoko**
**Meinyu-Shataku 403, 2985, Kamitsuruma**
**Sagamihara-shi, Kanagawa-ken(JP)**
Inventor: **Negi, Tahee**
**2-1-311, Kugenuma-Higashi**
**Fujisawa-shi, Kanagawa-ken(JP)**

(74) Representative: **Vossius & Partner**
**Siebertstrasse 4 P.O. Box 86 07 67**
**D-8000 München 86(DE)**

(54) Novel acinetobacter calcoaceticus and novel biosurfactant.

(57) A novel Acinetobacter calcoaceticus strain produces a polysaccharide type biosurfactant having emulsifying capacity in high yield, reduces litmus, is negative to oxidases, produces pale brown substances when it is cultured in an agar culture medium, and has an ability of reducing nitrates and of denitrification. The biosurfactant produced by the novel microorganism has emulsifying properties superior to the known biosurfactant produced by a known Acinetobacter calcoaceticus strain RAG-1. In addition, the strain makes it possible to produce the biosurfactant on an industrial scale.

EP 0 401 700 A2

# Novel Acinetobacter calcoaceticus and Novel Biosurfactant

The present invention relates to novel microorganisms which produce detergents having excellent capacity for emulsifying oils or the like (biosurfactant; hereinafter referred to as "BSF") as well as a novel BSF and a method for preparing the novel BSF.

Natural fat and oil type surfactants and petrochemical surfactants have widely been employed as soaps, synthetic detergents and emulsifying agents. On the other hand, the development of new surfactants having various functions comparable to or superior to those achieved by the conventional ones has recently been desired and as a result substances which have surface active properties and are produced by microorganisms have recently attracted special interest.

For instance, Japanese unexamined published Patent Application (hereinafter referred to as "J.P. KOKAI") No. Sho 55-112201 discloses microorganisms belonging to the genus Acinetobacter which produce BSF's and Petroleum Fermentations Co., Ltd. has already started industrial production of Emulsan which is a BSF produced by Acinetobacter calcoaceticus RAG-1 (ATCC 31012) disclosed in the foregoing Patent. Emulsan is used as a detergent for oil reservoirs of tankers. In addition, Sophorolipid (produced by Kao Corporation) which is a BSF produced by Torulopsis bombicola has already been put to practical use in certain kinds of products. Moreover, spiculisporic acid (available from IWATA CHEMICALS, INC.) produced by Penicillium spiculisporum has also been industrially produced.

In addition to the foregoing examples, there are known BSF's produced by a variety of microorganisms. Examples of such BSF's include glycolipid type ones such as trehalose lipids produced by Rhodococcus erythropolis and microorganisms belonging to genus Nocardia sp., sophorolipids produced by Torulopsis bombicola and ustilagic acid produced by Ustilago maydis; fatty acid type ones such as spiculisporic acid produced by Penicillium spiculisporum ; biopolymer type ones such as polysaccharide-lipid complexes or protein-lipid-polysaccharide complexes produced by Candida tropicalis or Corynebacterium hydrocarboclastus.

However, these BSF's are presently no match at all in cost for other synthetic surfactants and, contrary to expectation, they have not yet been put to practical use.

Accordingly, the primary object of the present invention is to provide novel microorganisms which can produce novel functional surfactants.

Another object of the present invention is to provide a novel BSF having excellent emulsifying ability.

A further object of the present invention is to provide a method for preparing the novel BSF having excellent ability of emulsifying oils or the like.

Various screenings were conducted for microorganisms which can produce polysaccharide type BSF's having excellent emulsifying capacity in high yield over wide natural areas. It was found that a certain species belonging to Acinetobacter calcoaceticus produces polysaccharide type BSF's having properties superior to those of Emulsan in a culture medium in high yield.

The present invention provides novel species belonging to Acinetobacter calcoaceticus which can produce a polysaccharide type BSF, the polysaccharide type BSF being able to reduce litmus, being negative to oxidases, producing pale brown substances when it is cultured in agar mediums, having an ability of reducing nitrates and of denitrification and having high emulsifying ability.

According to another aspect of the present invention, there is provided a novel BSF characterized in that:

(i) it consists of a polymer having a molecular weight of 1,000,000 to 5,000,000 and comprising neutral sugars, uronic acids, amino sugars and proteins which are bound to each other as constituents,

(ii) the neutral sugars constitute from 60 to 85 wt % of the polymer,

(iii) the neutral sugars comprise glucose, monnose and galactose in a weight ratio of 100:(30~100):-(40:~150),

(iv) the uronic acids constitute from 7 to 25 wt % of the polymer,

(v) the uronic acids comprise glucuronic acid and galacturonic acid in a weight ratio of 100:(60~20), and

(vi) the amino sugars and the proteins constitute the balance of the polymer, the ability of the surfactant to emulsify liquid paraffin being 40 to 60%.

According to a further aspect of the present invention, there is also provided a method for preparing the aforesaid novel BSF which comprises the step of culturing the foregoing Acinetobacter calcoaceticus in a culture medium.

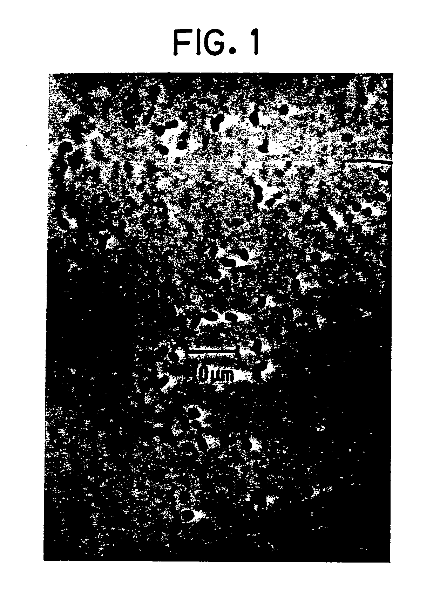

Fig. 1 is a light microscopic photograph illustrating the morphological characteristics of the novel microorganism of the present invention Acinetobacter calcoaceticus strain 217.

The present invention will be described in more detail below.

The novel microorganism of the present invention which belongs to genus Acinetobacter and has high BSF-producing ability is Acinetobacter calcoaceticus Species 217 which is deposited with Fermentation Research Institute (FRI) under the accession No. BP-2905 and its biological characteristic properties will be detailed below.

The determination of the characteristic biological properties and classification thereof were carried out according to the methods disclosed in "Bergey's Manual of Determinative Bacteriology", 1974, 8th edition; and the Screening Table of R.E. Gordon (1987). The determination of the range of the optimum growth temperature condition as a function of temperature and pH was performed using a temperature gradient biophotorecorder.

## A. Morphological Properties

When it was cultured at 30° C for 2 days on a broth-agar medium, the following morphological properties were observed.

1) Shape and Size of the Cell: short bacillus having a size of 1.7 to 1.9 $\mu$m × 2.6 to 2.7 $\mu$m;
2) It possesses polymorphism (from sphere to rod);
3) It has no motility;
4) It has no spore;
5) Gram-staining: negative;
6) Resistance to acid: negative;

## B. Cultivability

1) It grows at pH 7.0 and forms circular, flat, entire colonies. The surface of these colonies is smooth and glossy; the periphery thereof is pale brown; and the center is translucent and is pale brown.

2) Broth Agar Slant Culture

It grows at pH 7.0 on belt-shaped regions on the culture medium and forms glossy colonies which get cream color or pale brown. The culture medium gets pale brown due to the microorganism within one week or so.

3) Broth Liquid Culture

It grows at pH 7.0, but does not form pellicle.

4) Broth Gelatin Stab Culture

The culture medium is slightly liquefied at pH 7.0.

5) Litmus • Milk:

It slightly reduces litmus, but does not cause coagulation of caseinogen.

## C. Physiolosical Characteristics

1) Reduction of nitrates: positive
2) Denitrification reaction: positive
3) Methyl Red (MR) Test: negative
4) Voges-Proskauer (VP) Test: positive
5) Indole production: positive
6) Hydrogen sulfide production: positive
7) Starch hydrolysis: negative
8) Use of citric acid: It uses citric acid in Christensen's medium.
9) Use of inorganic nitrogen: It uses nitrates, but not ammonium salts.
10) Generation of dyes: It does not form any dye.
11) Urease: positive
12) Oxidase: negative
13) Catalase: positive
14) Growth temperature range: from 20 to 37° C; the optimum growth temperature: about 28 to 30° C.
15) Growth pH range: from 6.0 to 9.6; the optimum growth pH: about 6.8.
16) Behavior against oxygen: aerobic

17) Oxidation-Fermentation (O-F) Test: fermentation

18) Ornithine decarboxylation: negative

19) Arginine dihydrolase: negative

20) Lysine decarboxylation: positive

21) Extracellular DNase Test: negative

22) Assimilation of hydrocarbons: (Tween series)

Tween 40: positive

Tween 60: positive

Tween 80: negative

23) Generation of acids and gases from sugars:

| Sugar | Acid | Gas |
|---|---|---|
| L-arabinose | - | - |
| D-xylose | - | - |
| D-glucose | + | + |
| D-mannose | - | - |
| D-fructose | + | - |
| D-galactose | - | - |
| malt sugar (maltose) | + | - |
| cane sugar (sucrose) | + | - |
| trehalose | + | - |
| D-sorbit | - | - |
| D-mannit | + | - |
| inosit | - | - |
| glycerin | - | - |
| starch | - | - |
| +: generated | | |
| -: not generated. | | |

D. Other Miscellaneous Characteristics

1) The polysaccharide type polymers secreted by the strain 217 are novel biosurfactants exhibiting excellent emulsifying capacity.

2) This substance forms a thin film when a water or a water-alcohol solution (or a suspension) is applied to a substrate and then the solvent is removed therefrom.

The foregoing properties can be summarized as folows: this strain is a short bacillus which is positive to catalase test, is aerobic and gram-negative and shows polymorphism. Therefore, this strain was judged to be a bacillus belonging to Acinetobacter calcoaceticus.

A light microscopic photograph illustrating the shape (morphology) of the strain 217 is shown in Fig. 1.

Thus, the strain seems to belong to Acinetobacter calcoaceticus. Therefore, to differentiate the strain and other bacilli of genus Acinetobacter, the bacteriological properties of this strain is compared with those for Acinetobacter calcoaceticus RAG-1 (ATCC 31012) discovered by E. Rosenberg. The growth temperature of the strain of this invention is highly consistent with that for the strain RAG-1, but the strain reduce litmus, whereas the known strain cannot reduce the same. Regarding the oxidase test, the strain is negative to the test, while the known strain is positive thereto. Moreover, the strain produces substances which get pale brown on the broth agar culture medium, while the known strain does not produce such substances. Further, the strain exhibits high capacity of reducing nitrates and of denitrification, but the known strain does not exhibit such characteristic properties. Thus, the strain of the present invention can clearly be distinguished from the known one.

As has been discussed above, the strain differs from the known Acinetobacter calcoaceticus RAG-1. However, it is reasonable to judge that the strain is analogous to Acinetobacter calcoaceticus in the light of the foregoing bacteriological properties. The results of the comparison between them are summarized in the following Table I.

4

EP 0 401 700 A2

Table I

| Bacteriological Properties | Strain 217 | Strain RAG-1 |
|---|---|---|
| Size of Cell (μm) | 1.7~1.9 x 2.6~2.7 | 1.2~1.4 x 3.2~3.4 |
| Reduction of nitrates | + | - |
| Denitrification | + | - |
| Growth Temp. range ($^\circ$ C) | 20 to 37 | 26 to 40 |
| Optimum Temp. range ($^\circ$ C) | 28 to 30 | 30 to 37 |
| Growth pH range | 6.9 to 9.6 | 7.0 to 10.0 |
| Optimum pH range | around 6.8 | around 7.8 |
| Sugar sources from which acids are generated | glucose trehalose fructose maltose cane sugar mannit | in addition to those listed in the column of strain 217, arabinose, xylose galactose, glycerin. |
| Resistance to sodium chloride | does not grow in the presence of 5% NaCl | grows even in the presence of 10% NaCl |
| Reduction of litmus | + | - |
| Oxidase | - | + |
| Coloration of culture medium | + | - |

The surface active substances produced by the strain can be recovered by, for instance, the following method. First, the strain is cultured in a culture medium containing carbon sources, nitrogen sources and inorganic salts, then neutralized and heat-treated. Thereafter, the bacterial cells are removed by centrifugation, the resulting culture supernatant is condensed and/or dialyzed, 0.5 to 10 volumes of ethanol are added to the condensate or dialyzate with stirring and then the mixture is allowed to stand. The resulting precipitates are recovered and dried. The sample thus obtained is called BSF 217E. In this respect, the solvent to be added in this stage is not restricted to ethanol and hydrophilic solvents such as methanol and acetone may be used. In addition, the amount of the solvent to be added at this stage is not also critical so far as it is enough to sufficiently form precipitates. BSF 217E is purified by, for instance, the removal of proteins and the resulting purified product is named BSF 217.

The novel biosurfactant of the presnt invention is characterized in that:

(i) it consists of a polymer having a molecular weight of 1,000,000 to 5,000,000, preferably 2,800,000 to 3,700,000 (by HPLC) and comprising neutral sugars, uronic acids, amino sugars and proteins which are bound to each other as constituents,

(ii) the neutral sugars constitute from 60 to 85 wt %, preferably 65 ot 75 wt % of the polymer,

(iii) the neutral sugars comprise glucose, monnose and galactose in a weigh ratio of 100:(30~100):-(40:~150), preferably 100:(40~60): (50~100),

(iv) the uronic acids constitute from 7 to 25 wt %, preferably 10 to 18 wt % of the polymer,

5

(v) the uronic acids comprise glucuronic acid and galacturonic acid in a weight ratio of 100:(60~20), peferably 100:(50~30) and

(vi) the amino sugars and the proteins constitute the balance of the polymer,

(vii) the ability of the surfactant to emulsify liquid paraffin is 40 to 60 % by the following method: 10mℓ of a 0.5% by weight aqueous solution of each sample was mixed with 10 mℓ of liquid paraffin in a graduated test tube, vigorously shaken and allowed to stand at room temperature for 30 minutes, followed by determination and recording of the volume of the aqueous phase and the calculation of the emulsifying power on the basis of the following equation:

(10 - the volume of the aqueous phase)/10 X 100 (%)

(viii) c. m. c. of the surfactant is 0.1 to 0.5 wt % determined by a dye-solubilization method suing Oil Yellow,

(ix) viscosity of the surfactant is 10 to 50 cps where the viscosity of a 0.5 % by weight aqueous solutin of each sample is determined, using a Brookfield rotational viscometer, at 25 °C and a number of rotaion of 30 rpm,

(x) surface tension of a 0.5 % by weight aqueous solution of the surfactant (at room temperature) is 50 to 73 dyn/cm.

On the other hand, the representative constituents of Emulsan are as follows:

| | |
|---|---|
| D-galactosamine | 20 to 30 wt % |
| hexosaminouronic acid | 33.3 wt % |
| D-glucose | 5.2 wt % |
| fatty acid esters | 15.0 wt % |
| water | 12.7 wt % |
| ash | 3.5 % |

The main differences between BSF 217 and Emulsan in their constituents are as follows:

| | BSF 217 | Emulsan |
|---|---|---|
| mannose | large amount | no |
| glucose | large amount | no or less |
| galactosamine | no | large amount |

Thus, according to the present invention, there is provided a novel microorganism which can produces a BSF superior in emulsifying properties to the known BSF produced by Acinetobacter calcoaceticus RAG-1.

The present invention will be explained in more detail with reference to the following non-limiting working Examples.

Example 1

First, the inventors collected and isolated the strain of this invention from nature according to a screening method which will be described below.

1) Isolation of Polysaccharide-producing Bacteria

20 g of a yeast extract, 1 g of monopotassium phosphate and 1 g of magnesium sulfate were dissolved in 1ℓ of tap water and the pH value of the resulting solution was controlled to 7.0 by potassium hydroxide. After sterilization in an autoclave, glucose and n-decane which had been sterilized separately were added to the solution so that the concentrations thereof were 1% respectively. A liquid culture medium was dispensed in 7 mℓ into test tubes (∅ 16.5 x 165 mm), about 100 mg each of soil collected from various districts was added to a test tube and cultured at 30 °C for 5 days. Then the resulting culture media were inoculated on plates comprising the foregoing culture medium to which 1.5% agar was supplemented, incubated at 30 °C for 3 days and thus among the strains which grew on the plates, 109 strains having polysaccharide-producing ability were isolated.

### 2) Isolation of Polysaccharide Biosurfactant (BSF)-producing Bacteria

The emulsifying power of the resulting culture media of the polysaccharide-producing bacteria thus isolated was determined and was evaluated in terms of indices determined on the basis of the emulsifying power of EMULSAN to thus isolate polysaccharide type BSF-producing bacteria.

### 3) Isolation of Bacteria Having High Polysaccharide Type BSF-producing Ability

The selection for bacteria having high polysaccharide type BSF-producing ability was performed by evaluating their emulsifying and dispersing ability as well as productivity of the polysaccharide type BSF which was the most important factor for putting the present invention in practical use.

The numbers of strains which were screened through the aforesaid screening process are listed in the following Table II.

Table II

| Number of Soil Screened | 340 Places in the Country |
|---|---|
| 1) Number of bacteria collected | 319 strains |
| 2) Number of polysaccharide-producing bacteria | 109 strains |
| 3) Number of bacteria which produce polysaccharide type BSF in high yield | 1 strain (Ac. cal.) |

The microorganism which was screened through the foregoing screening processes from the natural world is the novel Acinetobacter calcoaceticus strain 217 (FERM BP-2905) which belongs to genus Acinetobacter, is excellent in emulsifying and dispersing ability and can produce polysaccharide type BSF in high yield.

These bacteria were collected from farmland in Arao City, Kumamoto Prefecture and the collection was carried out in 1987.

The emulsifying properties of the BSF 217E produced by the strain 217 were determined by the following method. BSF 217E was obtained by using the same culture medium and culture conditions as well as the method for preparing samples used in Example 3 explained below.

To a 100 mℓ volume Epton tube, there were added 30 mℓ of a 0.5% aqueous suspension of BSF 217E and 20 mℓ of salad oil, the resulting mixture was shaken up and down 100 times vigorously, allowed to stand for one day and then its emulsifying power was determined. The results obtained are listed in the following Table III together with the emulsifying properties of BSF 217E produced by known microorganisms. As seen from the results listed in this Table, the BSF produced by the microorganism of the present invention exhibits emulsifying power substantially higher than that of Emulsan. In Table III, the term BSF 217E means "BSF" produced by the microorganism of the present invention.

7

Table III

| BSF Sample | Emulsifying Power % (1 day after) | Name of Microorganism Producing the Corresponding BSF |
|---|---|---|
| Comp. Ex. | | |
| Gellan Gum | 2 | Pseudomonas erodea |
| Curdlan | 10 | Alcaligenes faecalis |
| pullulon | 2 | Aureobasidium pullulans |
| spiculisporic acid (Na salt at pH 7) | 0 | Penicillium spiculisporum |
| Emulsan | 32 | Acinetobacter calcoaceticus RAG-1 (ATCC No. 31012) |
| Present Invention | | |
| BSF 217E | 50 | Acinetobacter calcoaceticus strain 217 |

EP 0 401 700 A2

Example 2

Acinetobacter calcoaceticus strain 217 was cultured in the same medium and under the same culture conditions as those explained in the following Example 3, the resulting culture supernatant was concentrated, dialyzed and ethanol was added thereto to form precipitates. The precipitates thus obtained were recovered by centrifugation. The resulting crude sample was referred to as "BSF 217E".

The crude sample was further subjected to a hydrolase treatment, a treatment with a protein denaturing agent such as urea, or the like then purified by gel filtration, followed by drying and the resulting dried powder was used as a sample (BSF 217) for analyzing sugars contained therein. The analysis of the sugars was performed in the following manner.

Neutral sugar: phenol-sulfuric acid method.

Uronic acid: carbazole sulfuric acid method.

Amino sugars: Elson-Morgan method.

After hydrolyzation of the sample, BSF 217, with 2N hydrochloric acid, the sample was passed through an ion chromatography column to separate it into a neutral sugar fraction and an amino sugar fraction. The neutral sugar fraction was subjected to liquid chromatographic analysis (column: DIAION CDR-10 column; temp. 60 $^\circ$ C; reaction vessel 110 $^\circ$ C; measure wavelength 560 nm).

The results of the foregoing analysis indicate that the neutral sugars included in the sample, BSF 217, comprise mannose, galactose and glycose in a ratio of 1:1:2. The presence of mannose clearly indicates that the strain of the present invention substantially differs from the known strains since EMULSAN does not contain mannose at all. It can be concluded, from the foregoing results, that the sample, BSF 217, is a polysaccharide polymer which comprises a neutral sugar as a basic skeleton to which uronic acid, amino sugars, proteins or the like are bonded and that BSF 217 comprises about 70% of neutral sugars, about 13% of uronic acid, about 1% of amino sugars and the balance of proteins. In this connection, it was confirmed that the uronic acid comprises about 70% of glucuronic acid and about 30% of galacturonic acid and that 99% or more of the amino sugars are occupied by glucosamine.

Example 3

The molecular weight of the sample, BSF 217, obtained in Example 2 was determined in the following manner.

1) Molecular Weight Determination

The molecular weight was determined by a high-performance liquid-gel chromatograph (apparatus: Model 635A available from Hitachi, Ltd.) provided with columns PW6000 $_{XL}$ and PW3000 $_{XL}$ (available from TOSO CO., LTD.; developing solution: 0.1M phosphate buffer (pH 6.8); standard substance: polyethylene oxide).

As a result, the molecular weight of BSF 217 was found to be about 3,200,000.

The molecular weight of EMULSAN was likewise determined and found to be about 2,500,000.

2) BSF-productivity of the Strain 217

This strain was cultured under the following conditions in a culture medium detailed below and thus 10.3 g of BSF per 1 $\ell$ of the culture medium was recovered. The strain RAG-1 was cultured in the same manner and 5.3 g/$\ell$ of EMULSAN was recovered.

(Culture Medium; Growth Conditions)

To a 2$\ell$ volume SAKAGUCHI flask, there was added a culture medium (pH 7.15) which comprised 0.75% of dipotassium phosphate, 0.23% of monopotassium phosphate, 0.08% of ammonium sulfate, 0.07% of magnesium sulfate heptahydrate and 0.1% of a yeast extract (available from ORIENTAL YEAST CO., LTD.), then the culture medium was sterilized and glycose and sucrose which had been separately sterilized were added to the culture medium so that the concentrations thereof were 1% and 3% respectively. The strain of the present invention which had been precultured was aseptically inoculated into the culture medium and was subjected to a shaking culture at 30 ° C for 3 days.

(Method for preparing Sample)

After 3 days, the culture medium was neutralized, then heat treated at 100° C for 30 minutes and the bacterial cells were removed by centrifugation. The culture supernatant separated was concentrated, then dialyzed against water to remove low molecular weight substances, ethanol was added to the resulting dialyzate from which the low molecular weight substances had been removed, and the resulting mixture was allowed to stand at 4 ° C to obtain precipitates of BSF. The BSF was recovered as Sample, BSF 217E.

As has been discussed above in detail, the strain of the present invention exhibits BSF-producing ability higher than that of the control strain RAG-1.

Analysis of BSF 217E

The sample, BSF 217E, obtained above was extracted with water to separate it into a water-soluble fraction and a water-insoluble fraction. The water-soluble fraction accounted for 35% of the total and the water-insoluble fraction accounted for 65%. As seen from the result of quantitative analysis of sugars, proteins or the like, BSF 217E has a polysaccharide structure which comprises neutral sugars as principal components as well as uronic acid and amino sugars and to which proteins are bonded. The results of analysis of these fractions are as follows:

| Fraction | Total amount of Sugar | Uronic acid | Protein |
|---|---|---|---|
| | ($\mu$g/mg) | ($\mu$g/mg) | ($\mu$g/mg) |
| Water-soluble | 665 | 131 | 100 |
| Water-insoluble | 280 | 62 | 500 |

*: Both fractions contained about 1% of amino sugars.

Please note that the water-insoluble material is estimated to be a precipitate wherein polysaccharides are incorporated into protains which are modified and insolubilized by ethanol added in a method for preparing sample mentioned above.

Example 4

Quality of BSF 217 obtained in Example 3 or BSF 217E obtained in Example 2 was compared with that of EMULSAN. The results obtained are listed below.

Determination of Emulsifying Power

10 mℓ of a 0.5% by weight aqueous solution of each sample was mixed with 10 mℓ of liquid paraffin in a graduated test tube, vigorously shaken and allowed to stand at room temperature (for 15, 30 and 60 minutes), followed by determination and recording of the volume of the aqueous phase and the calculation of the emulsifying power on the basis of the following equation:

(10 - the volume of the aqueous phase)/10 X 100 (%)
The results thus obtained are summarized in Table IV given below.

Table IV

| Sample | cmc (%) | Emulsifying Power (%) | | |
|---|---|---|---|---|
| | | 15 min. | 30 min. | 60 min. |
| Emulsan | 0.3 | 5 | 5 | 0 |
| BSF 217E | 0.4 | 70 | 55 | 35 |
| BSF 217 | -- | 65 | 50 | 35 |
| cmc: Critical micelle concentration (determined by a dye-solubilization method using Oil Yellow). | | | | |

## Solubility in Solvent

BSF 217E is soluble in water and insoluble in solvents such as ethanol, ether and acetone.

## Viscosity

The viscosity of a 0.5% by weight aqueous solution of each sample was determined, using a Brookfield rotational viscometer, at 25 $^\circ$ C and a number of rotation of 30 rpm.

| Sample | Viscosity (cps) |
|---|---|
| BSF 217E | 10 |
| BSF 217 | 15 |
| Emulsan | 20 |

## Surface Tension

The surface tension of a 0.5% by weight aqueous solution of each Sample was determined at room temperature by a hanging plate method. The results thus obtained are summarized in Table given below.

| Sample | Surface Tension (dyn/cm) |
|---|---|
| BSF 217E | 72 |
| BSF 217 | 65 |
| Emulsan | 53 |

## Claims

1. Acinetobacter calcoaceticus which produces a polysaccharide type biosurfactant having an emul-

11

sifying capacity; reduces litmus; is negative to oxidases; produces pale brown substances when it is cultured in an agar culture medium; and is able to reduce nitrates and to denitrify.

2. The Acinetobacter calcoaceticus of claim 1, which is Acinetobacter calcoaceticus strain 217 (FERM BP-2905).

3. A method for preparing a biosurfactant characterized in that:

(i) it is a polymer having a molecular weight of 1,000,000 to 5,000,000 and comprising neutral sugars, uronic acids, amino sugars and proteins which are bound to each other as constituents,

(ii) the neutral sugars constitute from 60 to 85 wt % of the polymer,

(iii) the neutral sugars comprise glucose, mannose and galactose in a weight ratio of 100:(30~100):- (40:~150),

(iv) the uronic acids constitute from 7 to 25 wt % of the polymer,

(v) the uronic acids comprise glucuronic acid and galacturonic acid in a weight ratio of 100:(60~20), and

(vi) the amino sugars and the proteins constitute the balance of the polymer, the ability of the surfactant to emulsify liquid paraffin being 40 to 60%,

said method comprising the step of culturing, in a culture medium, Acinetobacter calcoaceticus which produces said polysaccharide type biosurfactant having an emulsifying capacity; reduces litmus; is negative to oxidases; produces pale brown substances when it is cultured in an agar culture medium; and is able to reduce nitrates and to denitrify.

4. The method of claim 3 wherein the Acinetobacter calcoaceticus is Acinetobacter calcoaceticus strain 217 (FERM BP-2905)

5. The method of claim 3 or 4, wherein the culturing of Acinetobacter calcoaceticus is carried out at a temperature of 20 to 30° C.

6. The method of claim 5 wherein the temperature is 28 to 30° C.

7. The method of any one of claims 3 to 6, wherein the culturing of Acinetobacter calcoaceticus is carried out at a pH ranging from 6.9 to 9.5.

8. The method of claim 7 wherein the pH is about 6.8.

9. The method of claim 3 or 4, wherein the culturing of Acinetobacter calcoaceticus is carried out at a temperature of 20 to 37 °C and a pH ranging from 6.9 to 9.6.

10. A biosurfactant characterized in that:

(i) it is a polymer having a molecular weight of 1,000,000 to 5,000,000 and comprising neutral sugars, uronic acids, amino sugars and proteins which are bound to each other as constituents,

(ii) the neutral sugars constitute from 60 to 85 wt % of the polymer,

(iii) the neutral sugars comprise glucose, mannose and galactose in a weigh ratio of 100:(30~100):- (40:~150),

(iv) the uronic acids constitute from 7 to 25 wt % of the polymer,

(v) the uronic acids comprise glucuronic acid and galacturonic acid in a weight ratio of 100:(60~20), and

(vi) the amino sugars and the proteins constitute the balance of the polymer, the ability of the surfactant to emulsify liquid paraffin being 40 to 60%.

11. A composition containing the biosurfactant according to claim 10.

12. The composition according to claim 11, which is a soap, a detergent or an emulsifying agent.

FIG. 1